# EUROPEAN PATENT SPECIFICATION

(11) **EP 3 377 180 B1**
(45) Date of publication and mention of the grant of the patent: **19.06.2019**
(21) Application number: 16782288.1
(22) Date of filing: 19.10.2016
(51) Int. Cl.: A61Q 5/00, A61K 8/49, A61K 8/11, A61K 8/35, A61K 8/92, A61K 9/00, A61K 9/50, A61K 47/08, A61K 47/44, A61K 9/06, A61K 8/84, A61K 8/73

(54) **CLIMBAZOLE MICROCAPSULE AND HAIR CARE COMPOSITION COMPRISING SURFACTANT AND CLIMBAZOLE**
CLIMBAZOLMIKROKAPSELN UND HAARPFLEGEZUSAMMENSETZUNG MIT TENSID UND CLIMBAZOL
MICROCAPSULE DE CLIMBAZOLE ET COMPOSITION DE SOIN CAPILLAIRE RENFERMANT UN TENSIOACTIF ET DU CLIMBAZOLE

(30) Priority: 17.11.2015 EP 15195054; 07.07.2016 EP 16178499
(43) Date of publication of application: 26.09.2018
(73) Proprietor: Unilever PLC, London Greater London EC4Y 0DY (GB); Unilever NV, 3013 AL Rotterdam (NL)
(72) Inventor: FERGUSON, Paul, Wirral Merseyside CH63 3JW (GB); GOULSBRA, Amanda, Marie, Wirral Merseyside CH63 3JW (GB); JONES, Christopher, Clarkson, Wirral Merseyside CH63 3JW (GB); MEALING, David, Richard, Arthur, Wirral Merseyside CH63 3JW (GB); MULKERRIN, George, Patrick, Wirral Merseyside CH63 3JW (GB); PARKER, Andrew, Philip, Wirral Merseyside CH63 3JW (GB)
(74) Representative: Chisem, Janet
(86) International application number: PCT/EP2016/075110
(87) International publication number: WO 2017/084826

(56) References cited:
- WO-A1-2014/173659
- WO-A2-2007/071325

## Description

### TECHNICAL FIELD

This invention relates to a microcapsule comprising climbazole, to a process to make the microcapsule comprising climbazole and to anti-dandruff hair care compositions. In particular compositions comprising surfactant and climbazole as anti-dandruff active.

### BACKGROUND

Climbazole is a racemate composed of equimolar amounts of (R)- and (S)-climbazole with IUPAC name (RS)-1-(4-Chlorophenoxy)-1-imidazol-1-yl-3,3-dimethylbutan-2-one and CAS registry number 38083-17-19. It is a topically applied antifungal agent used to treat human fungal skin infections. It may be included in over the counter anti-dandruff shampoo formulations and skin treatment compositions to treat dandruff and eczema where the cause is a fungal infection.

At an inclusion level of 1 wt% in anti-dandruff shampoo compositions comprising cleaning surfactant it has been shown that only around 0.05 wt% of the included climbazole stays on the scalp after shampooing. The vast majority is washed away due to a combination of poor deposition and the affinity of climbazole for the surfactant that is rinsed away.

It is taught in WO 00/66072 (P&G) that bioavailability and coverage of the anti-dandruff active are more predictive of anti-dandruff efficacy than mere deposition of the active on the hair or scalp. This document proposes measurement of efficacy by measuring inhibition of *Malassezia* furfur (*M*. Furfur) growth on agar contact plates.

It has been proposed to improve efficacy by delivering climbazole in encapsulated form, from a microcapsule.

WO 07/071325 (Unilever) discloses polymeric carrier particles comprising a pharmaceutical, skin or hair active, which can be climbazole (Examples 14, 15 and 16). Climbazole was incorporated into polystyrene latex particles via an aqueous phase. We have now found that this type of particle suffers from the twin problems of low climbazole inclusion and poor release of the climbazole from the particle due to its capsule architecture.

EP 1 702 674 (Cognis) discloses encapsulation of active ingredients and their use. Antidandruff agents can be encapsulated (paragraph [0011]) and these can include climbazole (p6 [0024]).

WO 12/138696 (P&G) discloses that there can be a problem of poor deposition of microcapsules from a shampoo because the presence of the surfactant helps to wash the microcapsules away. The proposed solution to obtain increased deposition of polyacrylate microcapsules is to use an anionically charged microcapsule in combination with a cationic deposition polymer. The microcapsules are given an anionic charge by coating them with an anionic emulsion. A suitable anionic emulsifier comprises a copolymer of acrylic acid and butyl acrylate. The perfume examples use emulsion polymerisation with perfume dispersed in an oil phase. On page 6 line 33 and page 7 line 1 there is a disclosure of a benefit agent, such as an anti-dandruff agent, which can be an azole. However, there is no indication of how such a benefit agent would be incorporated into a microcapsule.

WO 14/064121 (Unilever) discloses use of a chitosan salt as a deposition aid, at the outside of a microcapsule particle, to improve delivery of a benefit agent from the particle to a substrate, particularly hair. Deposition is improved compared with the same particle without the chitosan deposition aid on it. The particle can deliver a benefit agent which can be climbazole (page 8 line 1). Core-shell encapsulate particles are preferred (page 10 line 7), and suitable particles are melamine/urea-formaldehyde encapsulates (page 11 line 10). Some examples show deposition onto hair of fluorescer carrying polystyrene latex particles with chitosan deposition aid. Other examples show deposition onto hair of melamine formaldehyde core shell particles is possible when they have the chitosan deposition aid attached. There is no disclosure about how climbazole would have been loaded into the melamine formaldehyde capsules and no examples used climbazole.

There remains a need for an improved microcapsule suitable for delivery of climbazole to scalp with high efficiency and efficacy from a hair care composition comprising surfactant.

### SUMMARY OF THE INVENTION

According to the present invention there is provided a core shell microcapsule wherein the liquid core of the microcapsule comprises solvent and climbazole dissolved in the solvent in a weight ratio of at most 8:1 solvent: climbazole, preferably at most 5:1 solvent: climbazole.

Preferably the shell is a synthetic polymer material. Preferably the shell is water-insoluble and the liquid contents of the microcapsule are released by rupture of the microcapsule after deposition onto scalp or hair; usually after the microcapsule has become dry. Advantageously the shell has, fixed to its outside, a deposition aid, to increase the deposition and retention of the microcapsule on scalp and/or hair, especially hair roots.

Without wishing to be bound by theory it is believed that the localised high concentration of solvent assists the released climbazole solution to penetrate sebum and so improves anti-dandruff (*M*. Furfur) efficacy. We have found that the solvent should have the ability to dissolve climbazole at a level of at least 1 part by weight climbazole to 5 parts by weight solvent. Preferred solvents comprise perfume components, more preferably they comprise a significant portion of perfume mid notes or low notes to give low volatility so that the dissolved climbazole remains in solution after rupture of the microcapsule. Thus the solvent system should avoid volatile solvents whose use causes the climbazole to crystallise out of the solution in less than 1 hour at 25°C. The cLog P of the solvent should preferably be greater than 1.8, more preferably greater than 2, and most preferably greater than 3.

Also according to the present invention there is provided a process to manufacture microcapsules comprising climbazole the process comprising the steps of:
a) Dissolving climbazole in solvent to form an oil phase;
b) Mixing the oil phase with an oil soluble first component of a shell forming polymerization system;
c) Dispersing the mixture from step b in an aqueous phase which either contains, or has added to it, a water soluble second component of a shell forming polymerization system;
d) Forming microcapsules by reaction of the first and second components of the polymerization system to encapsulate the climbazole dissolved in the perfume; and
e) Optionally separating the formed microcapsules from the aqueous phase.

The polymerisation process may use a Pickering emulsion type process. Such a process is taught in WO 2008/145547. Shell chemistries known to the skilled person that are based on Pickering emulsion processes are melamine formaldehyde, urea formaldehyde and melamine glyoxal. Alternatively, the polymerisation process may be done by interfacial polymerisation. A suitable process for making polyurea microcapsules is taught in US2013/330292. Shell chemistries known to the skilled person that are based on interfacial polymerisation are polyurea formed by reaction of polyisocyanates and polyamines; polyurethane formed by reaction of polyisocyanates and polyols; mixtures of polyurethane and polyurea; polyamides, polyesters and inorganic shells such as silicas.

Advantageously a deposition aid is added either before or during step (d) to covalently bond to the surface of the microcapsules. Alternatively, a spacer may be bonded to the surface of the microcapsules and a deposition aid then reacted onto the spacer. Polyethylene glycol is a suitable spacer. Preferred deposition aids are selected from the group consisting of: Grafted dextran, HPC (and the other hydrophobically modified polysaccharides), chitosan salts and peptides.

Also according to the present invention there is provided a hair shampoo comprising surfactant and at least 0.1 wt% of a core shell microcapsule comprising a polymer shell and a core comprising climbazole and perfume in a ratio of at least 1:8, preferably at least 1:5. Preferably, the shell comprises melamine formaldehyde or polyurea.

Increased deposition and release of climbazole lead to increased anti-dandruff efficacy. Anti *M.* Furfur effect has been shown for ruptured microcapsules through a Zone of Inhibition test.

Frequently anti-dandruff shampoos comprise more than one anti-dandruff active. Zinc pyrithione (ZPT) is known to affect *M.* Furfur in a different way from climbazole, so the combination of climbazole with ZPT is desirable. The problem has been that the less reliable deposition of climbazole from a composition containing both free climbazole and free ZPT means that the ratio of the two deposited materials is unpredictable and the synergistic efficacy is hence unreliably delivered. On the other hand the known climbazole containing microcapsules contain low concentrations of climbazole and do not deliver it in an efficacious manner; so again the synergy of the ZPT and the climbazole is not obtained in practice.

A preferred shampoo comprises ZPT in addition to the climbazole microcapsules.

### DETAILED DESCRIPTION OF THE INVENTION

### The solvent

It is essential that the solvent can dissolve climbazole efficiently or the number of microcapsules needed in a hair care composition will become intolerably high. The minimum dissolving power for the present invention is such that at most 8 times, preferably at most 5 times as much solvent is used as climbazole is dissolved. Once dissolved it is important that the climbazole stays in solution as this aids transport of the climbazole once the microcapsule fractures and delivers its liquid core payload. Preferred solvents comprise perfume components and in that case they will comprise at least 50 wt% mid notes and low notes to give low volatility so that the dissolved climbazole remains in solution after rupture of the microcapsule. Thus the solvent system should avoid volatile solvents whose use causes the climbazole to crystallise out of an open test solution in less than 1 hour at 25°C. The cLog P of the solvent should preferably be greater than 1.8, more preferably greater than 2, and most preferably greater than 3. A suitable solvent that can be used in admixture or alone is heptanone.

### The perfume

Surprisingly, we have found that not all perfume raw materials are capable of dissolving climbazole at sufficient levels to be useful in the invention. Furthermore, whilst highly volatile materials often make good solvents these are unsuited to the present application because the solvent will evaporate before the climbazole has been transported to where it is needed. Preferred perfume solvents are selected from those comprising at least 50 wt% low and mid notes and having a cLogP of at least 1.8, preferably at least 2, more preferably at least 3. Preferably the perfume is selected from the group consisting of Beta-lonone, Clove oil, Eugenol and Cyclocitral. Proprietary perfumes and perfume mixtures suitable for use in anti-dandruff shampoos are also suitable. It is acceptable to warm the perfume a little to increase climbazole solubility. However, the climbazole should stay in solution post manufacture of the microcapsule or else the benefit of delivery from solution will be partially lost and efficacy will drop.

### The anti-dandruff active

The anti-dandruff agent is climbazole. Climbazole is a racemate composed of equimolar amounts of (R)- and (S)-climbazole with IUPAC name (RS)-1-(4-Chlorophenoxy)-1-imidazol-1-yl-3,3-dimethylbutan-2-one and CAS registry number 38083-17-19. This antidandruff active inhibits the enzyme lanosterol 14 a-demethylase; the enzyme necessary to convert lanosterol to ergosterol. Depletion of ergosterol in fungal membrane disrupts the structure and many functions of fungal membrane leading to inhibition of fungal growth.

The anti-dandruff agent may be solely climbazole or it may comprise a mixture of climbazole with at least one secondary anti-dandruff agent. The secondary anti-dandruff agents may be either encapsulated or non-encapsulated. If they are encapsulated they may be co-encapsulated with the climbazole, or they may be contained in a second type of microcapsule. A preferred secondary anti-dandruff active is zinc pyrithione.

Preferably, the total anti-dandruff agent is present in the composition in an amount of from 0.1 to 5 wt%, more preferably from 0.1 to 2 wt%.

### Climbazole level

The level of climbazole in the microcapsule should be as high as possible without it coming out of solution. For the preferred solvent systems the level per particle will typically be at least twice that of the latex particles of the prior art. This means that the amount of delivery system is reduced compared with that prior art. Preferred climbazole levels are at least 20 wt% of the particle solids (shell, solvent and climbazole).

Preferably, the climbazole is present at from 0.01 to 1 wt% of the composition, more preferably from 0.1 to 0.6 wt% and most preferably from 0.1 to 0.5 wt% of the composition.

### The microcapsule wall material

The microcapsule has a shell wall. Preferably, the wall material is a synthetic polymer material. Preferred shell materials melamine formaldehyde, urea formaldehyde and melamine glyoxal. Also preferred is polyurea formed by reaction of polyisocyanates and polyamines; polyurethane formed by reaction of polyisocyanates and polyols; mixtures of polyurethane and polyurea; polyamides, polyesters and even inorganic shells such as silicas.

The most preferred wall material is selected from melamine formaldehyde and polyurea.

Advantageously the microcapsule wall comprises at most 20 wt% of the weight of the microcapsules.

### Microcapsule size

The diameter of the microcapsules is preferably at least 5 microns. The diameter should be less than 50 microns as if it is larger than that the particles become visible to the naked eye. Preferably, microcapsule diameters lie in the range, 5 to 20 micron, for example 10 to 15 micron.

### Deposition aid

A deposition aid with affinity for hair root or scalp may be grafted on to the outside of the microcapsule shell. Preferred deposition aids and grafting methods for the preferred shell materials are taught elsewhere and are known to the skilled worker. Most preferred are: grafted dextran; HPC (and the other hydrophobically modified polysaccharides); and chitosan salts. WO 14/064121 discloses use of a chitosan salt as a deposition aid for increasing deposition of microcapsules onto hair, WO 13/026656 discloses use of dextran as a deposition aid for use to deposit microcapsules from a hair shampoo and WO 13/026657 discloses use of HPMC and HEMC as deposition aids to assist deposition of microcapsules from a hair shampoo.

### The surfactant system

The composition may be in any common product form used as a hair care product to treat hair. Preferably, it is a rinse off composition and most preferably, it is an anti-dandruff shampoo composition.

The composition may comprise any of the ingredients commonly found in hair care products depending on the product form.

For example, when the composition is a shampoo it will comprise a surfactant system including at least one cleansing surfactant suitable for use in shampoos. When it is a composition which aims to provide conditioning benefit, it will comprise a conditioning active. Suitable conditioning actives include fatty alcohols, silicones and cationic surfactants.

Examples of suitable anionic cleansing surfactants are the alkyl sulphates, alkyl ether sulfates, alkaryl sulfonates, alkanoyl isethionates, alkyl succinates, alkyl sulfosuccinates, alkyl ether sulfosuccinates, N-alkyl sarcosinates, alkyl phosphates, alkyl ether phosphates, and alkyl ether carboxylic acids and salts thereof, especially their sodium, magnesium, ammonium and mono-, di-and triethanolamine salts. The alkyl and acyl groups generally contain from 8 to 18, preferably from 10 to 16 carbon atoms and may be unsaturated. The alkyl ether sulfates, alkyl ether sulfosuccinates, alkyl ether phosphates and alkyl ether carboxylic acids and salts thereof may contain from 1 to 20 ethylene oxide or propylene oxide units per molecule.

Typical anionic cleansing surfactants for use in compositions of the invention include sodium oleyl succinate, ammonium lauryl sulfosuccinate, sodium lauryl sulfate, sodium lauryl ether sulphate, sodium lauryl ether sulphosuccinate, ammonium lauryl sulfate, ammonium lauryl ether sulfate, sodium dodecylbenzene sulfonate, triethanolamine dodecylbenzene sulfonate, sodium cocoyl isethionate, sodium lauryl isethionate, lauryl ether carboxylic acid and sodium N-lauryl sarcosinate.

Preferred anionic surfactants are the alkyl sulfates and alkyl ether sulfates. These materials have the respective formulae ROSO₃M and R-O(C₂H₄O)ₓSO₃M, wherein R is alkyl or alkenyl of from 8 to 18 carbon atoms, x is an integer having a value of from about 1 to about 10, and M is a cation such as ammonium, alkanolamines, such as triethanolamine, monovalent metals, such as sodium and potassium, and polyvalent metal cations, such as magnesium, and calcium. Most preferably, R has 12 to 14 carbon atoms, in a linear rather than branched chain.

Preferred anionic cleansing surfactants are selected from sodium lauryl sulfate and sodium lauryl ether sulfate(n)EO, (where n is from 1 to 3); more preferably sodium lauryl ether sulfate(n)EO, (where n is from 1 to 3); most preferably sodium lauryl ether sulfate1EO.

Preferably the level of alkyl ether sulfate is from 0.5 wt% to 25 wt% of the total composition, more preferably from 3 wt% to 18 wt%, most preferably from 6 wt% to 15 wt% of the total composition.

The total amount of anionic cleansing surfactant in compositions of the invention generally ranges from 0.5 wt% to 45 wt%, more preferably from 1.5 wt% to 20 wt%.

Compositions of the invention may contain non-ionic surfactant. Most preferably, non-ionic surfactants are present in the range 0 to 5 wt%.

Non-ionic surfactants that can be included in compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups. Alkyl ethoxylates are particularly preferred. Most preferred are alkyl ethoxylates having the formula R-(OCH₂CH₂)ₙOH, where R is a C₁₂₋₁₅alkyl chain and n is 5 to 9.

Other suitable non-ionic surfactants include mono- or di-alkyl alkanolamides. Examples include coco mono- or di-ethanolamide and coco mono-isopropanolamide.

Further non-ionic surfactants which can be included in shampoo compositions of the invention are the alkyl polyglycosides (APGs). Typically, APG is one which comprises an alkyl group connected (optionally via a bridging group) to a block of one or more glycosyl groups. Preferred APGs are defined by the following formula:

RO-(G)ₙ

wherein R is a branched or straight chain alkyl group which may be saturated or unsaturated and G is a saccharide group. R may represent an alkyl chain with a mean length of from about C₅ to about C₂₀. Most preferably R represents an alkyl chain with a mean length of from about C_{9.5} to about C_{10.5}. G may be selected from C5 or C6 monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies from about 1.1 to about 2. Most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex BASF (DeWolf).

Other sugar-derived non-ionic surfactants which can be included in compositions of the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C10-C18 N-(3-methoxypropyl) glucamide.

Amphoteric or zwitterionic surfactant can be included in an amount ranging from 0.5 wt% to about 8 wt%, preferably from 1 wt% to 4 wt% of the total shampoo composition.

Examples of amphoteric or zwitterionic surfactants include alkyl amine oxides, alkyl betaines, alkyl amidopropyl betaines, alkyl sulfobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulfopropyl betaine, lauryl betaine, cocamidopropyl betaine and sodium cocoamphoacetate.

A particularly preferred amphoteric or zwitterionic surfactant is cocamidopropyl betaine. Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

Particularly preferred compositions comprise a surfactant system comprising: 10 to 20 wt% of the composition sodium lauryl sulphate or sodium lauryl ether sulphate (n) EO, (where n ranges from 1 to 3); 0.5 to 5 wt% of the composition cocamidopropylbetaine; and 0.5 to 5 wt% of the composition sodium cocoamphoacetate or sodium lauryl cocoamphoacetate.

### Other ingredients

The compositions may also include one or more of the following non-essential ingredients:

### pH adjusters

The pH of the compositions is preferably in the range of from 5 to 8, more preferably in the range of from 6 to 7 e.g., 6.5. The pH of the compositions can be adjusted using alkaline agents (such as sodium hydroxide, for example) or acidic agents (such as citric acid) as is well-known in the art.

### Cationic Polymer

A cationic polymer is a preferred ingredient in the hair care compositions according to the invention, for enhancing conditioning performance of the compositions.

The cationic polymer may be a homopolymer or be formed from two or more types of monomers. The molecular weight of the polymer will generally be between 5 000 and 10 000 000, typically at least 10 000 and preferably in the range 100 000 to about 2 000 000. The polymers will have cationic nitrogen containing groups such as quaternary ammonium or protonated amino groups, or a mixture thereof.

The cationic nitrogen-containing group will generally be present as a substituent on a fraction of the total monomer units of the cationic polymer. Thus, when the polymer is not a homopolymer it can contain spacer non-cationic monomer units. Such polymers are described in the CTFA Cosmetic Ingredient Directory, 3rd edition. The ratio of the cationic to non-cationic monomer units is selected to give a polymer having a cationic charge density in the required range.

Suitable cationic conditioning polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quatemization.

The cationic conditioning polymers can comprise mixtures of monomer units derived from amine - and/or quaternary ammonium-substituted monomer and/or compatible spacer monomers. Suitable cationic conditioning polymers include, for example:
a) copolymers of 1-vinyl-2-pyrrolidine and 1-vinyl-3-methyl-imidazolium salt (e.g. chloride salt), referred to in the industry by the Cosmetic, Toiletry, and Fragrance Association, (CTFA) as Polyquatemium-16. This material is commercially available from BASF Wyandotte Corp. (Parsippany, NJ, USA) under the LUVIQUAT tradename (e.g. LUVIQUAT FC 370);
b) copolymers of 1-vinyl-2-pyrrolidine and dimethylaminoethyl methacrylate, referred to in the industry (CTFA) as Polyquatemium-11. This material is available commercially from Gaf Corporation (Wayne, NJ, USA) under the GAFQUAT tradename (e.g., GAFQUAT 755N);
c) cationic diallyl quaternary ammonium-containing polymers including, for example, dimethyldiallyammonium chloride homopolymer and copolymers of acrylamide and dimethyldiallylammonium chloride, referred to in the industry (CTFA) as Polyquatemium 6 and Polyquatemium 7, respectively;
d) mineral acid salts of amino-alkyl esters of homo-and co-polymers of unsaturated carboxylic acids having from 3 to 5 carbon atoms, (as described in U.S. Patent 4,009,256);
e) cationic polyacrylamides (as described in WO95/22311).

Other cationic conditioning polymers that can be used include cationic polysaccharide polymers, such as cationic cellulose derivatives, cationic starch derivatives, and cationic guar gum derivatives. Suitably, such cationic polysaccharide polymers have a charge density in the range from 0.1 to 4 meq/g.

Cationic polysaccharide polymers suitable for use in compositions of the invention include those of the formula:

A-O-[R-N⁺(R¹)(R²)(R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquatemium 10. Another type of cationic cellulose includes the polymeric quaternary ammonium salts of hydroxyethyl cellulose reacted with lauryl dimethyl ammonium-substituted epoxide, referred to in the industry (CTFA) as Polyquatemium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimonium chloride (commercially available from Rhone-Poulenc in their JAGUAR trademark series).

Examples are JAGUAR C13S, which has a low degree of substitution of the cationic groups and high viscosity. JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, and JAGUAR 162 which is a high transparency, medium viscosity guar having a low degree of substitution.

Preferably the cationic conditioning polymer is selected from cationic cellulose and cationic guar derivatives. Particularly preferred cationic polymers are JAGUAR C13S, JAGUAR C15, JAGUAR C17 and JAGUAR C16 and JAGUAR C162. The presence of a cationic polymer is believed to enhance deposition of encapsulates comprising grafted HPC deposition aid.

The cationic conditioning polymer will generally be present in compositions of the invention at levels of from 0.01 to 5, preferably from 0.05 to 1, more preferably from 0.08 to 0.5 wt% of the composition.

When cationic conditioning polymer is present in a hair care composition according to the invention, it is preferred if the copolymer is present as emulsion particles with a mean diameter (D_{3,2} as measured by light scattering using a Malvern particle sizer) of 2 micrometres or less.

Hair care compositions of the invention are preferably aqueous, i.e. they have water or an aqueous solution or a lyotropic liquid crystalline phase as their major component. Suitably, the composition will comprise from 50 to 98 wt%, preferably from 60 to 90 wt% water based on the total weight of the composition.

### Silicone

The anti-dandruff hair compositions may additional comprise from 0.1 to 10 wt%, preferably from 0.1 to about 8 wt%, more preferably from about 0.3 to about 5 wt% of a silicone.

Preferred suitable silicones may include polyalkyl siloxanes, polyaryl siloxanes, polyalkylaryl siloxanes, polyether siloxane copolymers, amino silicones and mixtures thereof.

The silicone may be present as the free silicone oil, or in the form of a silicone emulsion.

Preferably the silicone is present in the form of a silicone emulsion, more preferably an aqueous surfactant stabilized emulsion of silicone particles having a number average particle diameter ranging from 10 to 1,000 nm, most preferably from about 100 to about 500 nm.

Amino silicones are often formulated in hair compositions. Amino silicones are silicones containing at least one primary amine, secondary amine, tertiary amine or a quaternary ammonium group. High molecular weight silicone gums can also be utilized. Another useful type are the crosslinked silicone elastomers such as Dimethicone/Vinyl/ Dimethicone Crosspolymers (e.g. Dow Coming 9040 and 9041).

Examples of suitable pre-formed silicone emulsions include emulsions DC2-1766, DC2-1784, DC-1785, DC-1786, DC-1788 and microemulsions DC2-1865 and DC2-1870, all available from Dow Corning. These are all emulsions or microemulsions of dimethiconol. Also suitable are amodimethicone emulsions such as DC939 (from Dow Coming) and SME253 (from GE Silicones).

### Suspending Agent

Preferably, the hair care composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent, if included, will generally be present in a hair care composition of the invention at levels of from 0.1 to 10 wt%, preferably from 0.5 to 6 wt%, more preferably from 0.9 to 4 wt% based on the total weight of the composition.

### Non-silicone Oily Conditioning Components

Compositions according to the present invention may also comprise a dispersed, non-volatile, water-insoluble oily conditioning agent.

This component will be dispersed in the composition in the form of droplets, which form a separate, discontinuous phase from the aqueous, continuous phase of the composition. In other words, the oily conditioning agent will be present in the shampoo composition in the form of an oil-in-water emulsion.

By "insoluble" is meant that the material is not soluble in water (distilled or equivalent) at a concentration of 0.1 percent (w/w), at 25 °C. Suitably, the D3 2 average droplet size of the oily conditioning component is at least 0.4, preferably at least 0.8, and more preferably at least 1 micro m. Additionally, the D3 2 average droplet size of the oily conditioning component is preferably no greater than 10, more preferably no greater 8, more preferably no greater than 5, yet more preferably no greater than 4, and most preferably no greater than 3.5 µm.

The oily conditioning agent may suitably be selected from oily or fatty materials, and mixtures thereof.

Oily or fatty materials are preferred conditioning agents in the shampoo compositions of the invention for adding shine to the hair and also enhancing dry combing and dry hair feel.

Preferred oily and fatty materials will generally have a viscosity of less than 5 Pa.s, more preferably less than 1 Pa.s, and most preferably less than 0.5 Pa.s, e.g. 0.1 Pa.s and under as measured at 25 degrees centigrade with a Brookfield Viscometer (e.g. Brookfield RV) using spindle 3 operating at 100 rpm.

Oily and fatty materials with higher viscosities may be used. For example, materials with viscosities as high as 65 Pa.s may be used. The viscosity of such materials (i.e. materials with viscosities of 5 Pa.s and greater) can be measured by means of a glass capillary viscometer as set out further in Dow Coming Corporate Test Method CTM004, July 20 1970.

Suitable oily or fatty materials are selected from hydrocarbon oils, fatty esters and mixtures thereof.

Hydrocarbon oils include cyclic hydrocarbons, straight chain aliphatic hydrocarbons (saturated or unsaturated), and branched chain aliphatic hydrocarbons (saturated or unsaturated). Straight chain hydrocarbon oils will preferably contain from about 12 to about 30 carbon atoms. Branched chain hydrocarbon oils can and typically may contain higher numbers of carbon atoms. Also suitable are polymeric hydrocarbons of alkenyl monomers, such as C2 to C6 alkenyl monomers. These polymers can be straight or branched chain polymers. The straight chain polymers will typically be relatively short in length, having a total number of carbon atoms as described above for straight chain hydrocarbons in general. The branched chain polymers can have substantially higher chain length. The number average molecular weight of such materials can vary widely, but will typically be up to about 2000, preferably from about 200 to about 1000, more preferably from about 300 to about 600.

Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used. Exemplary branched-chain isomers are highly branched saturated or unsaturated alkanes, such as the permethyl-substituted isomers, e.g., the permethyl-substituted isomers of hexadecane and eicosane, such as 2, 2, 4, 4, 6, 6, 8, 8-dimethyl-10-methylundecane and 2, 2, 4, 4, 6, 6-dimethyl-8-methylnonane, sold by Permethyl Corporation. A further example of a hydrocarbon polymer is polybutene, such as the copolymer of isobutylene and butene. A commercially available material of this type is L-14 polybutene from Amoco Chemical Co. (Chicago, III., U.S.A.).

Particularly preferred hydrocarbon oils are the various grades of mineral oils. Mineral oils are clear oily liquids obtained from petroleum oil, from which waxes have been removed, and the more volatile fractions removed by distillation. The fraction distilling between 250 degrees centigrade to 300 degrees centigrade is termed mineral oil, and it consists of a mixture of hydrocarbons ranging from C16H34 to C21 H 4. Suitable commercially available materials of this type include Sirius M85 and Sirius M125, all available from Silkolene.

Suitable fatty esters are characterised by having at least 10 carbon atoms, and include esters with hydrocarbyl chains derived from fatty acids or alcohols, e.g., monocarboxylic acid esters, polyhydric alcohol esters, and di- and tricarboxylic acid esters. The hydrocarbyl radicals of the fatty esters hereof can also include or have covalently bonded thereto other compatible functionalities, such as amides and alkoxy moieties, such as ethoxy or ether linkages. Monocarboxylic acid esters include esters of alcohols and/or acids of the formula R'COOR in which R' and R independently denote alkyl or alkenyl radicals and the sum of carbon atoms in R' and R is at least 10, preferably at least 20.

Specific examples include, for example, alkyl and alkenyl esters of fatty acids having aliphatic chains with from about 10 to about 22 carbon atoms, and alkyl and/or alkenyl fatty alcohol carboxylic acid esters having an alkyl and/or alkenyl alcohol-derived aliphatic chain with about 10 to about 22 carbon atoms, benzoate esters of fatty alcohols having from about 12 to 20 carbon atoms.

The monocarboxylic acid ester need not necessarily contain at least one chain with at least 10 carbon atoms, so long as the total number of aliphatic chain carbon atoms is at least 10. Examples include isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate. Di- and trialkyl and alkenyl esters of carboxylic acids can also be used. These include, for example, esters of C4 to C8 dicarboxylic acids such as C7 to C22 esters (preferably C1 to C9) of succinic acid, glutaric acid, adipic acid, hexanoic acid, heptanoic acid, and octanoic acid. Examples include diisopropyl adipate, diisohexyl adipate, and diisopropyl sebacate. Other specific examples include isocetyl stearoyl stearate, and tristearyl citrate.

Polyhydric alcohol esters include alkylene glycol esters, for example ethylene glycol mono and di-fatty acid esters, diethylene glycol mono- and di-fatty acid esters, polyethylene glycol mono- and di-fatty acid esters, propylene glycol mono- and di-fatty acid esters, polypropylene glycol monooleate, polypropylene glycol monostearate, ethoxylated propylene glycol monostearate, polyglycerol polyfatty acid esters, ethoxylated glyceryl monostearate, 1,3-butylene glycol monostearate, 1,3-butylene glycol distearate, polyoxyethylene polyol fatty acid ester, sorbitan fatty acid esters, polyoxyethylene sorbitan fatty acid esters and mono-, di-and triglycerides.

Particularly preferred fatty esters are mono-, di- and triglycerides, more specifically the mono-, di-, and tri-esters of glycerol and long chain carboxylic acids such as C7 to C22 carboxylic acids. A variety of these types of materials can be obtained from vegetable and animal fats and oils, such as coconut oil, castor oil, safflower oil, sunflower oil, cottonseed oil, com oil, olive oil, cod liver oil, almond oil, avocado oil, palm oil, sesame oil, peanut oil, lanolin and soybean oil. Synthetic oils include triolein and tristearin glyceryl dilaurate.

Specific examples of preferred materials include cocoa butter, palm stearin, sunflower oil, soyabean oil and coconut oil. The oily or fatty material is suitably present at a level of from 0.05 to 10, preferably from 0.2 to 5, more preferably from about 0.5 to 3 wt%.

The compositions of this invention preferably contain no more than 3 wt% of a styling polymer, more preferably less than 1 percent of a styling polymer, preferably contain less than 0.1 wt% styling polymer, and optimally are free of styling polymer.

In hair treatment compositions containing a conditioning agent, it is preferred that a cationic polymer also be present.

### Adjuvants

The compositions of the present invention may also contain adjuvants suitable for hair care. Generally such ingredients are included individually at a level of up to 2, preferably up to 1 wt% of the total composition.

Among suitable hair care adjuvants, are:
(i) natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts. A particularly preferred combination of natural hair root nutrients for inclusion in compositions of the invention is isoleucine and glucose. A particularly preferred amino acid nutrient is arginine.
(ii) hair fibre benefit agents. Examples are: ceramides, for moisturising the fibre and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratoires Serobiologiques.

### Minor ingredients

The compositions may also include other ingredients for enhancing performance and/or consumer acceptability. Such ingredients include fragrance (encapsulated or free or both), colorants, dyes and pigments, pearlescers or opacifiers, viscosity modifiers, stabilisers and preservatives. A suitable preservative system comprises Sodium Benzoate and Sodium Salicylate with pH adjustment using Sodium hydroxide and Citric Acid H₂O. An alternative preservation system comprising formaldehyde comprises: MIT and DMDM Hydantoin.

### Product forms

The hair care compositions may suitably be shampoos, conditioners, sprays, mousses, gels, waxes or lotions. Particularly preferred product forms are shampoos, post-wash conditioners (leave-in and rinse-off) and hair treatment products such as hair essences. Rinse off products are preferred and shampoos are particularly preferred.

Preferably, the compositions are free of, or substantially free of, hair styling polymer.
The compositions are preferably formulated as compositions for the treatment of hair and subsequent rinsing.

A particularly preferred hair care composition is a shampoo composition. The total amount of surfactant in shampoo compositions of the invention (including any co-surfactant, and/or any emulsifier) is generally from 5 to 30 wt%, preferably from 10 to 25 wt%, more preferably from 15 to 20 wt% of the composition.

The invention will now be further described with reference to the following non-limiting examples.

### EXAMPLES

### Example 1 - Choice of solvent

The manufacturer of Crinipan® AD (the climbazole tested) says in their published literature that: "Crinipan® AD [...] has good compatibility with perfume oils and other commonly used hair care raw materials and is soluble in alcohol, glycols, surfactants and certain perfume oils".

A number of perfume components, perfume mixtures and oils considered to be safe to use on scalp were screened to evaluate their ability to solubilise climbazole. Because emulsion polymerisation also encapsulates the solvent an efficient solvent is needed to get a high loading of climbazole into a microcapsule.

We found that many of the oils and perfumes tested did not effectively solubilise the climbazole at the ratios tested, in particular: Ethyl Cinnamate, Anethole, Coconut oil, Eucalyptus oil and propylene glycol failed to solubilise it at any ratio tested.

Based on this screening work, Beta-lonone (5:1 ratio of solvent to climbazole), Clove oil (2:1), two proprietary perfume mixtures suitable for use in anti-dandruff shampoos (5:1) and (2:1) and Benzyl Acetate (5:1) were chosen as preferred solvents for climbazole. Benzyl acetate was not further tested because it was considered to be a top note perfume and as such too volatile to be used alone in the present invention.

### Example 2 - Melamine formaldehyde Microcapsule Synthesis

A number of melamine formaldehyde encapsulates, encapsulating the preferred climbazole in oil solutions from Example 1, were synthesised using the following method. The materials used in the encapsulation are given in Table 1.

**Table 1 - Materials used**

| **Material** | **Supplier** | **% Active** |
|---|---|---|
| Beta-lonone (solvent) | Aldrich | >99% |
| Formalin (Aqueous Formaldehyde) | Aldrich | 37% |
| Melamine | Aldrich | 100% |
| Sodium Chloride | Aldrich | 100% |
| Sodium Carbonate | Aldrich | >99.5% |
| Formic Acid | Aldrich | >99% |
| Crinipan® AD (Climbazole) | Symrise | 100% |

In a 300 ml reaction vessel 19.5 g of Formalin (37 wt% aq. formaldehyde) was added to 44 g of water. The pH was adjusted to 8.9 using 5% Sodium Carbonate. To this 10 g of melamine was added with 0.64 g of Sodium Chloride and stirred for 10 minutes at room temperature. The resulting solution was heated to 62°C and stirred until clear. This created a 23.2 wt% pre-polymer of trimethyloyl melamine in water.

4 g of Crinipan® AD was pre-dissolved in the solvent, in this case Beta-lonone (20 g) at a weight ratio of 5:1.

In a second 300 ml reaction vessel, 108.9 g of water was added and heated to 75 °C. 24.2 g of the pre-polymerwas added and the mixture pH adjusted to 4.1 using 10 wt% formic acid. After approximately 1 minute the solution became turbid. The solution was then agitated in a homogeniser at 10,000 rpm and 16.9 g of the Climbazole/Beta-lonone solution was added slowly over 30 seconds. The mixture was then further agitated for another 90 seconds. The reaction vessel was then returned to the overhead stirrer at 75°C for 3 hrs. After this time the mixture was cooled and adjusted to pH 7 using 5% Sodium Carbonate.

Other encapsulate samples were made using the same process and replacing the Beta-lonone with Clove Oil, or other perfume components, perfumes or oils.

### Example 3 - Zone of Inhibition Testing

Materials Used: Model Sebum Analogue consisted of 77% Captex 8000 (Glyceryl Trioctanoate) (Abitec) and 23% Squalene (Aldrich)

### Culture Preparation

1. Inoccula were prepared by adding loopfuls of culture from agar plate sub cultures of *M*. Furfur to sterile Tryptone in a bottle. The cultures were stirred for about 1 hour, prior to allowing them to settle for approximately 20 minutes.
2. The supernatant was carefully removed to another sterile bottle and the Optical Density in McFarland Units adjusted to 2.0 (approx. 1.2 E06 cfu/ml).
3. An inoculum was prepared from the original culture preparation by performing a 1/4 dilution (e.g. 2 ml culture to 6 ml Tryptone).

**Table 2 - Test Solutions**

| | Ratio of Climb: Oil | % encaps to give 0.1% | Solids | % slurry to give 0.1% | 10g Shampoo 10% hole | | |
|---|---|---|---|---|---|---|---|
| | | | | | Slurry | shampoo | water |
| Perfume 1 | 1:5 | 0.80% | 13.11% | 6.10% | 0.610 | 9 | 0.390 |
| Beta-Ionone | 1:5 | 0.80% | 12.86% | 6.22% | 0.622 | 9 | 0.380 |
| Clove oil | 1:1 | 0.27% | 12.82% | 2.11% | 0.211 | 9 | 0.789 |
| Perfume 2 | 1:5 | 0.80% | 11.92% | 6.71% | 0.671 | 9 | 0.329 |
| Perfume 3 | 1:2 | 0.40% | 12.91% | 3.10% | 0.310 | 9 | 0.690 |

Perfume 1 is a model perfume consisting of equal parts by weight of top notes, middle notes and base notes with composition: 11.3 wt% 1-acetate, 2-(1 ,1-dimethylethyl)-cyclohexanol, 1.6 wt% 1-(2,6,6-trimethyl-3-cyclohexen-1-yl)-2-buten-1-one, 6.7 wt% dodecanal, 6.7 wt% 4-(2,6,6- trimethyl-1-cyclohexen-1-yl)-3-buten-2-one, 6.7 wt% 4,7-Methano-1H-inden-6-ol, 3a,4,5,6,7,7a-hexahydro-, 6-acetate, 6.7 wt% 2-ethyl-4-(2,2,3-trimethyl-3-cyclopenten-1-yl)-2-buten-1-ol, 6.7 wt% 1-(1,2,3,4,5,6,7,8-octahydro-2,3,8,8-tetramethyl-2-naphthalenyl)-ethanone, 6.7 wt% 2-(phenylmethylene)-octanal, 6.7 wt% Oxacyclohexadecan-2-one, 6.7 wt% Benzeneacetic acid, 2-phenylethyl ester, 6.7 wt% 2-methyl-pentanoic acid, ethyl ester, 6.7 wt% octanal, 6.7 wt% 3,7-dimethyl-3-octanol, 6.7 wt% benzyl ethanoate, 6.7 wt% 3,7-dimethyl-, 3-acetate-1,6-octadien-3-ol. Perfumes 2 and 3 are commercially available perfumes suited to fabric conditioner applications with a balance of top notes, middle notes and base notes.

Solids level was measured by evaporating off the water either at 50°C or 120°C, depending on the fragility of the encapsulate.

The climbazole encapsulate slurries in Table 2 were added to the shampoo base given in Table 3. All the shampoos were calculated to have 0.1 wt% climbazole active. 1 g of each shampoo was then added to 3.9 g water and 0.1 g model sebum and 15 µl was added to filter papers in 6 repeats. Samples were then dried down in the oven at 45°C for half an hour to drive off the water and half of the samples were rubbed with a Teflon rod to simulate rupture.

**Table 3 - Shampoo**

| **INCI name** | **Function** | **wt%** |
|---|---|---|
| Sodium Laureth Sulfate | Primary Surfactant | 20 |
| Cocamidopropyl Betaine | Co-surfactant | 5.33 |
| Carbomer | Suspending agent | 0.6 |
| Sodium Hydroxide | pH adjuster | 0.3 |
| Citric Acid | pH adjuster | 0.06 |
| Methylisothiazolinone (MIT) | Preservative | 0.10 |
| Sodium chloride | Thickener | 0.5 |
| DMDM Hydantoin | Preservative | 0.3 |
| Polypropylene glycol | Viscosity adjuster | Up to 0.20 |
| Water | | to 100 |

### Zone of Inhibition (ZOI) Method

1. L&N agar plates were appropriately labelled.
2. 0.25 ml or 1 ml of quarter strength inoculum was pipetted onto the surface of each plate.
3. The inoculum was spread evenly over the whole surface of the plates using a sterile spreader.
4. Filter paper discs were added, in triplicate to the control plate and plates labelled 1 to 5 unruptured and left for 30 minutes in order to soak up any moisture from the agar.
5. 15 µl of each (diluted, unruptured) formulation were pipetted onto each of the 3 discs in the appropriate agar plate. The liquid was allowed to soak into the disc.
6. An additional disc was placed over the original discs in the control and 1 to 5 unruptured plates and the liquid allowed to soak into the additional discs.
7. The discs from the previously ruptured samples were added in triplicate to separate Leeming and Notman agar plates and allowed to absorb moisture from the plates.
8. The plates were sealed using Petriseal tape, taped together and placed in a plastic bag containing damp "Kimwipe" and incubated for 7 days at 30°C.
9. Any zone of inhibition observed was measured and recorded.

**Table 4 - ZOI results for perfume solvents**

| **Active** | **ZOI (mm)** | **ZOI (mm)** | **ZOI (mm)** | **Mean ZOI (mm)** | **SD** | **SE** |
|---|---|---|---|---|---|---|
| Control | 0 | 0 | 0 | 0.00 | 0 | 0 |
| Perfume 1 Unruptured | 0 | 0 | 0 | 0.00 | 0 | 0 |
| β-Ionone Unruptured | 0 | 0 | 0 | 0.00 | 0 | 0 |
| Clove Oil Unruptured | 2 | 1 | 1 | 1.33 | 0.58 | 0.33 |
| Perfume 2 Unruptured | 0 | 0 | 2 | 0.67 | 1.15 | 0.67 |
| Perfume 3 Unruptured | 0 | 0 | 0 | 0.00 | 0 | 0 |
| Perfume 1 Ruptured | 8 | 9 | 7 | 8.00 | 1.00 | 0.58 |
| β-Ionone Ruptured | 6 | 8 | 18 | 10.67 | 6.43 | 3.71 |
| Clove Oil Ruptured | 10 | 10 | 9 | 9.67 | 0.58 | 0.33 |
| Perfume 2 Ruptured | 9 | 8 | 8 | 8.33 | 0.58 | 0.33 |
| Perfume 3 Ruptured | 7 | 6 | 7 | 6.67 | 0.58 | 0.33 |

As expected the control shampoo base has no antimicrobial effect and the unruptured microcapsules had little or no antimicrobial effect vs M. Furfur. Whilst the ruptured microcapsules are significantly more antimicrobial than unruptured ones vs. M. Furfur, there are no significant differences in antimicrobial effect between the ruptured microcapsules using the different perfume solvent systems using this method.

### Example 4: Skin Deposition

Pig skin was cut into 50 x 50 mm sections, base washed with 14 % SLES solution and shaved. 0.5 g of shampoos as detailed in Table 3 and containing 1 wt% encap solids were applied to the pig skin. The skin was massaged with a finger for 30 seconds and then rinsed with 4 l/min 35°C tap water for a further 30 seconds. The skin was cut in half and placed in a 60 ml glass jar with 6 ml ethanol and extracted for 30 mins. This extract was then subjected to HPLC UV measuring the peaks at 220 nm and 280 nm and comparing to a calibration to give a level of ng climbazole per cm² skin.

Unencapsulated (free) climbazole deposition level was measured as 400 ng/cm², while the β-Ionone solvent encapsulates gave a deposition level of 750 ng/cm².

### Example 5: ZOI test COMPARING WITH PS LATEX

The efficacy of climbazole microcapsules on the growth of *M.* Furfur measured using Zone of Inhibition (ZOI) when the microcapsules had been ruptured by simple rubbing to simulate hair washing was tested both for the solvent containing core shell microcapsules according to the invention and the latex particles according to the prior art.

1gt of the shampoo of table 3 (with or without the microcapsule) was diluted with 3.9 g water and added to 0.1 g model sebum.

The prior art latex particles were made according to WO 07/071325.

| Comparative Example B | Latex (NO CBZ)(Polystyrene Chitosan) |
|---|---|
| Chitosan | 0.5 g |
| Water | 100 ml |
| Acetic Acid (36%) | 2.7 ml |
| Styrene | 2.5 ml |
| KPS | 50 mg |
| Expected Solids | 2.3% |
| Actual Solids | 1.8% |

### Method

The water was fully degassed for several hours by bubbling nitrogen. The reaction was carried out in 250 ml flask fitted with a magnetic stirrer bar, thermometer and nitrogen line. Chitosan (0.5 g) was added directly to fully degassed water (100 ml) under stirring at 800 rpm. Acetic acid solution (2.7 ml; 36%) prepared using degassed water was then added. The reaction mixture was heated at a pot temperature of 70°C under a blanket of nitrogen until a clear solution was obtained (approx. 20 to 30 min). Potassium persulphate KPS (50 mg) was then added in one portion and stirred for a few mins before the addition of styrene (2.5 ml). The reaction mixture was heated/stirred for a further 4 hours (after approx. 1 hour a white emulsion was observed). The styrene was used directly from the bottle (the inhibitor was not removed). The reactions were carried out in a single pot to ensure consistent nitrogen cover and thermal contact.

| Comparative Example C | Latex (+CBZ) (PS/Chitosan/CBZ) |
|---|---|
| Chitosan | 0.5 g |
| Water | 100 ml |
| Acetic Acid (36%) | 2.7 ml |
| Styrene | 2.5 ml |
| CBZ | 0.5 g |
| KPS | 50 mg |
| Expected Solids | 2.8% |
| Actual Solids | 2% |

The method for the Latex + CBZ material was as LATEX No CBZ except that CBZ (0.5g) was added at the same time as the potassium persulphate.

**Table 5**

| **Code** | **Sample** | **Ruptured mean ZOI (mm)** | **SE** |
|---|---|---|---|
| A | Free CBZ | 1.00¹ | 0.00 |
| B | Polystyrene/chitosan | 0.00 | 0.00 |
| C | PS/chitosan/CBZ | 3.67 | 0.33 |
| 1 | MF (as in E.g. 1: 5:1 Beta ionone) | 4.67 | 0.88 |

| | | | |
|---|---|---|---|
| ¹ Comparative Example A was free climbazole | | | |

The ZOI data given in table 5 supports two advantages of the microcapsules according to the invention. Firstly they can be used at lower levels due to their higher loadings of climbazole. Secondly the efficacy of the climbazole when the capsules are ruptured is significantly greater than the prior art microcapsules.

### Example 6: Polyurea microcapsule synthesis

Poly urea (PU) encapsulates, encapsulating climbazole in 2-heptanone, were synthesised using the following method. The materials used are given in Table 6. The method given is for microcapsule 6.2.

**Table 6 - Materials used**

| **Material** | **Supplier** | **% Active** |
|---|---|---|
| 2-Heptanone - (537683) | Aldrich | 99% |
| PMDI, Polymethylene polyphenyl isocyanate - (372986) | Aldrich | <=100% |
| Hexamethylenediamine (HMD) - (H11696) | Aldrich | 98% |
| Morwet D425, Napthalene sulphonate condensate | AkzoNobel | |
| Hydroxy propyl cellulose (HPC) (370K) | Aldrich | |

### Step 1

Preparation of carrier oil emulsion. Five grams of 2-heptanone and 1 g of climbazole was weighed out and agitated until fully dissolved. This solution was combined with 0.6 g of isocyanate (Aldrich, PMDI, Polymethylene polyphenyl isocyanate) to form the oil phase. In a separate vessel, a 10% surfactant solution (8g) was prepared by dissolving a sufficient amount of Morwet D-425 (Akzo Nobel) in deionised water. An amount of water (21.33 ml) was added to adjust the overall surfactant concentration to 3%. The oil phase was then emulsified into the aqueous phase to form the climbazole emulsion under shearing (Ultra Turrax T25, IKA) at 15000 rpm for two minutes.

### Step 2

Formation of the climbazole capsules. The climbazole emulsion prepared in step 1 was placed in a glass vessel to which 0.64 mL of a 40% hexamethylene diamine (HMDA) (Aldrich) was added over one minute under constant mixing at 200 to 300 rpm with a magnetic stirrer. The emulsion was stirred for 15 minutes.

### Step 3

Grafting of the deposition aid. 3.43 g of a 2% solution of Hydroxypropyl cellulose (HPC, 370K)(Aldrich)) was then added in two portions. The slurry was then cured at 55 °C for 3 to 5 hours.

**Table 7 - Examples of PU Encapsulates**

| **Chemical** | **6.1** | **6.2** | **6.3** | **6.4** |
|---|---|---|---|---|
| | BARE | 1% HPC | 3% HPC | 5% HPC |
| | weight (g) | weight (g) | weight (g) | weight (g) |
| Hept/CBZ (5:1) | 6.00 | 6.00 | 6.00 | 6.00 |
| PMDI | 0.60 | 0.60 | 0.60 | 0.60 |
| HMDA (40%) | 0.64 | 0.64 | 0.64 | 0.64 |
| Morwet D425 (10%) | 8.00 | 8.00 | 8.00 | 8.00 |
| HPC (2%) | 0.00 | 3.43 | 10.29 | 17.14 |
| Water adjustment | 24.76 | 21.33 | 14.47 | 7.61 |
| Check Sum (25g) | 40.00 | 40.00 | 40.00 | 40.00 |

### Example 7 - Deposition of polyurea microcapsules onto hair

Two CBZ containing anti-dandruff shampoo formulations were prepared based on the shampoo base given in Table 8. To one of the formulations was added free CBZ at 0.25 % and to the other PU encapsulated CBZ with 3% HPC as per example 6.3 to give the same level of CBZ inclusion.

**Table 8**

| **Ingredient** | **wt%** |
|---|---|
| SLES | 14.00 |
| Carbopol 980 | 0.60 |
| Tego betaine (CAPB) | 1.60 |
| Silicone | 2.20 |
| Jaguar C14S | 0.20 |
| Perfume and minor ingredients | 2.35 |
| CBZ | 0.25 |
| Water | To 100 |

Five virgin hair switches each weighing 5 g and 254 mm length were washed together. 2.5 g of shampoo was applied and washed for 30 seconds. Then the hair was rinsed for 30 seconds. Then another 2.5 g of shampoo was applied and washed for 30 seconds. Then the hair was rinsed for 30 seconds. The switches were then dried in a drying cabinet for 30 minutes at 50 °C, then removed and placed in separate 60 ml glass jars with their metal clip removed. 20 g of ethanol was added and the jars rolled for 60 minutes. After an hour the sample was filtered through a 1.2 |jm filter into a HPLC vial and sealed. The amount of CBZ deposited onto the switches and thereafter extracted was then determined by HPLC and is expressed in table 9 in terms of µg / cm²

**Table 9**

| Sample | Deposition (µg/cm²) |
|---|---|
| Free | 0.0068 |
| PU-HPC (3%) | 0.047 |

### Example 8: Skin Deposition

Four CBZ containing formulations were prepared by post dosing to the composition given in table 8 encapsulated climbazole or free climbazole equal 0.25% climbazole inclusion. One encap was a MF similar to example 1 but using heptanone as the solvent. The other two were both PU; one with an added HPC deposition aid.

Pig skin was cut into 3 x 3 cm² pieces after being washed and shaved previously. The skin was massaged with a gloved finger for 30 seconds and then rinsed under a pump water supply supplying 75 ml over 10 seconds and rubbed with the glove finger during this process. The skin was left to dry in the fume cupboard to reduce excess water, then an extraction ring was applied to the skin surface and 3 ml of ethanol added. This was rubbed around the skin surface using a Teflon rod then the solution removed for analysis via HPLC. The results of the HPLC determination of deposition are given in table 10.

**Table 10**

| Sample | Deposition (µg/cm²) | Confidence |
|---|---|---|
| Free | 0.5 | 0.43 |
| 6.1 PolyUrea | 1.27 | 1.27 |
| 6.2 PolyUrea-HPC (1%) | 1.36 | 0.7 |
| Melamine Formaldehyde | 1.25 | 0.93 |

The deposition from both types of encapsulate shells was greater than the free CBZ and the addition of the HPC deposition aid to the PU shell significantly improved the deposition further.

## Claims

1. A core shell microcapsule wherein the liquid core of the microcapsule comprises solvent and climbazole dissolved in the solvent in a weight ratio of at most 8:1, preferably at most 5:1 solvent: climbazole.

2. A microcapsule according to claim 1 wherein the solvent comprises perfume.

3. A microcapsule according to claim 2 wherein the perfume comprises at least 50 wt% of perfume mid notes or low notes and no volatile materials so that the climbazole does not crystallise out of solution when left standing for up to 1 hour at 25°C.

4. A microcapsule according to any preceding claim wherein the cLog P of the solvent is greater than 1.8, preferably greater than 2, more preferably greater than 3.

5. A microcapsule according to any preceding claim wherein the shell is made by interfacial polymerisation.

6. A microcapsule according to any preceding claim wherein the shell comprises melamine formaldehyde or polyurea.

7. A microcapsule according to any preceding claim wherein the shell is water-insoluble and the liquid contents of the microcapsule are released by rupture of the microcapsule after deposition onto scalp or hair after the microcapsule has become dry.

8. A microcapsule according to any preceding claim wherein the shell has fixed to its outside surface a deposition aid that increase the deposition and retention of the microcapsule on scalp and / or hair, especially hair roots.

9. A microcapsule according to claim 8 wherein the deposition aid is hydroxyl propyl cellulose

10. A process to manufacture microcapsules according to any one of claims 1 to 9 the process comprising the steps of:
a) Dissolving climbazole in solvent to form an oil phase;
b) Mixing the oil phase with an oil soluble first component of a shell forming polymerization system;
c) Dispersing the mixture from step b in an aqueous phase which either contains, or has added to it, a water soluble second component of a shell forming polymerization system;
d) Forming microcapsules by reaction of the first and second components of the polymerization system to encapsulate the climbazole dissolved in the solvent; and
e) Optionally separating the formed microcapsules from the aqueous phase.

11. A process according to claim 10 wherein a deposition aid is added either before or during step (d) to covalently bond to the surface of the microcapsules.

12. A hair care composition comprising surfactant and at least 0.1 wt% of a core shell microcapsule comprising a polymer shell and a core comprising climbazole and solvent wherein the climbazole is dissolved in the solvent in a weight ratio of at most 5:1 solvent.

13. A hair care composition according to claim 12 wherein the solvent consists essentially of perfume.

14. A hair care composition according to claim 12 or 13 wherein the amount of climbazole is from 0.1 to 0.5 wt% based on the hair care composition.

15. A hair care composition according to any one of claims 12 to 14 wherein the hair care composition is an anti-dandruff shampoo.

## Patentansprüche

1. Kern-Schale-Mikrokapsel, wobei der flüssige Kern der Mikrokapsel Lösungsmittel und Climbazol, gelöst in dem Lösungsmittel, in einem Gewichtsverhältnis von höchstens 8:1, vorzugsweise höchstens 5:1 von Lösungsmittel : Climbazol umfasst.

2. Mikrokapsel nach Anspruch 1, wobei das Lösungsmittel Parfüm umfasst.

3. Mikrokapsel nach Anspruch 2, wobei das Parfüm mindestens 50 Gew.-% Parfüm von mittlerer Note oder von niedrigen Noten und keine flüchtigen Materialien umfasst, so dass das Climbazol aus der Lösung nicht auskristallisiert, wenn man diese bis zu 1 Stunde bei 25°C stehen lässt.

4. Mikrokapsel nach irgendeinem vorhergehenden Anspruch, wobei der cLog P des Lösungsmittels größer als 1,8, vorzugsweise größer als 2, bevorzugter größer als 3 ist.

5. Mikrokapsel nach irgendeinem vorhergehenden Anspruch, wobei die Schale durch Grenzflächenpolymerisation hergestellt wird.

6. Mikrokapsel nach irgendeinem vorhergehenden Anspruch, wobei die Schale Melamin-Formaldehyd oder Polyharnstoff umfasst.

7. Mikrokapsel nach irgendeinem vorhergehenden Anspruch, wobei die Schale wasserunlöslich ist und der flüssige Inhalt der Mikrokapsel durch Bruch der Mikrokapsel nach Ablagerung auf der Kopfhaut oder dem Haar freigesetzt wird, nachdem die Mikrokapsel trocken geworden ist.

8. Mikrokapsel nach irgendeinem vorhergehenden Anspruch, wobei die Schale auf ihrer Außenfläche ein Ablagerungshilfsmittel fixiert aufweist, was die Ablagerung und Retention der Mikrokapsel auf der Kopfhaut und/oder dem Haar, insbesondere den Haarwurzeln, anhebt.

9. Mikrokapsel nach Anspruch 8, wobei das Ablagerungshilfsmittel Hydroxylpropylcellulose ist.

10. Verfahren zur Herstellung von Mikrokapseln nach irgendeinem der Ansprüche 1 bis 9, wobei das Verfahren die Schritte umfasst:
a) Auflösen von Climbazol in einem Lösungsmittel, um eine Öl-Phase zu bilden,
b) Mischen der Öl-Phase mit einem öllöslichen ersten Bestandteil eines Schale-bildenden Polymerisationssystems,
c) Dispergieren der Mischung vom Schritt b) in einer wässrigen Phase, die entweder einen wasserlöslichen zweiten Bestandteil eines Schale-bildenden Polymerisationssystems enthält oder welcher ihr zugefügt worden ist,
d) Ausbilden von Mikrokapseln durch Reaktion des ersten und zweiten Bestandteils des Polymerisationssystems, um das Climbazol, gelöst in dem Lösungsmittel, einzukapseln, und
e) optional Abtrennen der gebildeten Mikrokapseln aus der wässrigen Phase.

11. Verfahren nach Anspruch 10, wobei ein Ablagerungshilfsmittel entweder vor oder während dem/des Schritt(s) (d) zur kovalenten Bindung auf der Oberfläche der Mikrokapseln zugegeben wird.

12. Haarpflegezusammensetzung, umfassend Tensid und mindestens 0,1 Gew.-% einer Kern-Schale-Mikrokapsel, die eine polymere Schale und einen Kern, der Climbazol und Lösungsmittel enthält, umfasst, wobei das Climbazol in dem Lösungsmittel in einem Gewichtsverhältnis von höchstens 5:1 zum Lösungsmittel gelöst wird.

13. Haarpflegezusammensetzung nach Anspruch 12, wobei das Lösungsmittel im Wesentlichen aus Parfüm besteht.

14. Haarpflegezusammensetzung nach Anspruch 12 oder 13, wobei die Menge vom Climbazol von 0,1 bis 0,5 Gew.-%, bezogen auf die Haarpflegezusammensetzung, beträgt.

15. Haarpflegezusammensetzung nach irgendeinem der Ansprüche 12 bis 14, wobei die Haarpflegezusammensetzung ein Antischuppen-Shampoo enthält.

## Revendications

1. Microcapsule noyau enveloppe où le noyau liquide de la microcapsule comprend un solvant et du climbazole dissous dans le solvant dans un rapport en poids solvant : climbazole d'au plus 8:1, de préférence d'au plus 5:1.

2. Microcapsule selon la revendication 1 où le solvant comprend un parfum.

3. Microcapsule selon la revendication 2 où le parfum comprend au moins 50 % en poids de notes de coeur de parfum ou de notes de fond de parfum et aucune substance volatile de sorte que le climbazole ne cristallise pas dans la solution quand il est laissé au repos pendant jusqu'à 1 heure à 25°C.

4. Microcapsule selon l'une quelconque des revendications précédentes où le cLogP du solvant est supérieur à 1,8, de préférence supérieur à 2, de préférence encore supérieur à 3.

5. Microcapsule selon l'une quelconque des revendications précédentes où l'enveloppe est produite par polymérisation interfaciale.

6. Microcapsule selon l'une quelconque des revendications précédentes où l'enveloppe comprend du mélamine formaldéhyde ou de la polyurée.

7. Microcapsule selon l'une quelconque des revendications précédentes où l'enveloppe est insoluble dans l'eau et le contenu liquide de la microcapsule est libéré par rupture de la microcapsule après dépôt sur le cuir chevelu ou les cheveux après que la microcapsule soit devenue sèche.

8. Microcapsule selon l'une quelconque des revendications précédentes où l'enveloppe comporte, fixé à sa surface externe, un auxiliaire de dépôt qui augmente le dépôt et la rétention de la microcapsule sur le cuir chevelu et/ou les cheveux, en particulier les racines des cheveux.

9. Microcapsule selon la revendication 8 où l'auxiliaire de dépôt est l'hydroxypropylcellulose.

10. Procédé pour fabriquer des microcapsules selon l'une quelconque des revendications 1 à 9 le procédé comprenant les étapes de :
a) dissolution de climbazole dans un solvant pour former une phase huileuse ;
b) mélange de la phase huileuse avec un premier composant soluble dans l'huile d'un système de polymérisation de formation d'enveloppe ;
c) dispersion du mélange provenant de l'étape b dans une phase aqueuse qui contient, ou comporte, ajouté à elle, un second composant soluble dans l'eau d'un système de polymérisation de formation d'enveloppe ;
d) formation de microcapsules par réaction des premier et second composants du système de polymérisation pour encapsuler le climbazole dissous dans le solvant ; et
e) éventuellement séparation des microcapsules formées de la phase aqueuse.

11. Procédé selon la revendication 10 où un auxiliaire de dépôt est ajouté avant ou pendant l'étape (d) pour se lier de manière covalente à la surface des microcapsules.

12. Composition de soins capillaires comprenant un tensioactif et au moins 0,1 % en poids d'une microcapsule noyau enveloppe comprenant une enveloppe polymère et un noyau comprenant du climbazole et un solvant où le climbazole est dissous dans le solvant dans un rapport en poids d'au plus 5:1 de solvant.

13. Composition de soins capillaires selon la revendication 12 où le solvant consiste essentiellement en parfum.

14. Composition de soins capillaires selon la revendication 12 ou 13 où la quantité de climbazole est de 0,1 à 0,5 % en poids sur la base de la composition de soins capillaires.

15. Composition de soins capillaires selon l'une quelconque des revendications 12 à 14 où la composition de soins capillaires est un shampoing antipelliculaire.
